# EUROPEAN PATENT SPECIFICATION

(11) **EP 1 659 946 B1**
(45) Date of publication and mention of the grant of the patent: **14.04.2010**
(21) Application number: 04775170.6
(22) Date of filing: 10.08.2004
(51) Int. Cl.: A61B 7/02

(54) **STETHOSCOPE WITH PERSONALIZING-IDENTIFYING RING**
STETHOSKOP MIT PERSONALISIERTEM IDENTIFIKATIONSRING
STETHOSCOPE A BAGUE D'IDENTIFICATION ET DE PERSONNALISATION

(30) Priority: 14.08.2003 PL 36168603; 29.07.2004 PL 36934604
(43) Date of publication of application: 31.05.2006
(73) Proprietor: Brudkiewicz-Krysztof, Joanna Magdalena, 05-092 Lomianki (PL); Krysztof, Adam Rajmund, 05-092 Lomianki (PL); Krysztof, Michal Jan, 05-092 Lomianki (PL)
(72) Inventor: Brudkiewicz-Krysztof, Joanna Magdalena, 05-092 Lomianki (PL); Krysztof, Adam Rajmund, 05-092 Lomianki (PL); Krysztof, Michal Jan, 05-092 Lomianki (PL)
(74) Representative: Slominska-Dziubek, Anna
(86) International application number: PCT/PL2004/000064
(87) International publication number: WO 2005/016148

(56) References cited:
- US-A- 5 747 752
- US-A- 5 798 489
- US-A- 5 847 330
- US-A1- 2003 047 376

## Description

The present invention relates to a stethoscope head with distinguishing means for personalization and identification thereof, in particular a medical stethoscope head provided with a replaceable diaphragm portion and personalizing-identifying ring having such distinguishing means.

A medical stethoscope continuous to be a basic equipment both for doctors and graduate nurses, making the number of said stethoscopes enormous in every-day use. In connection with that, the problem of identifying a stethoscope and its user (owner) in health service institutions has emerged, especially when the stethoscopes come from one manufacturer.

As per observations of external appearance of the art known medical stethoscopes coming from different manufacturers, it is difficult to see any difference, between most of them, concerning their external shapes and basic details, and many of them look exactly the same, even in very fine elements, although manufacturers thereof are located in different and often very distant countries. It is a result of copying, striving for reduction of the production costs as well as using the uniformed elements, manufactured in long series by the specialized manufacturers. It especially applies to Y-shaped tubes, which join a head of the stethoscope with its headset, and to the elastic rings that fasten a diaphragm to a diaphragm cup, soft diaphragm covers and headframes.

A confusing similarity of stethoscopes leads to the considerable inconveniences resulting especially from the unintentional swap of stethoscopes by their users, which results from the difficulties to distinguish the official stethoscopes from the private ones, to identify the stethoscopes being the equipment of various departments of health service institutions, to identify the stethoscope by its individual user in the case it is accidentally placed next to the same model, etc. Besides, the companies, e.g. pharmaceutical companies, are not able to use medical stethoscopes for advertisement purposes and to brand them such that they would unequivocally differ, with an exemption of overprints on diaphragms or headset pipes, from the equipment used in the advertising campaigns of other companies. There also remains an unsatisfied demand directed to stethoscopes' manufacturers to unconventionally brand and/or decorate their products in relation to the competition's products or even to products of the same kind.

Attempts are known to distinguish stethoscopes by using various kinds of user-distinguishing or decorative elements. US200100188 and publication of the international patent application WO 01/13788 disclose the decorative covers having a form of sleeves which are put on the whole stethoscopes or on their parts which couple the medical stethoscope head with the headset imitating the animals' heads, whose purpose is to reduce children's stress related to medical examination.

Patent US 5920038 discloses medical stethoscope head comprising a diaphragm with graphical marking. The diaphragm is exchangeable inside the head and can be adjusted in relation to a given patient or user. This arrangement is designed mainly for examining the children and its intended purpose is to reduce stress and aversion to medical examination.

Publication US2004/0048539 discloses a cover of the medical stethoscope head having a form of an elastic net, preferably of knitted fabric, or elastic cover imitating an animal head, which is put on the stethoscope head. The task of a cover is to reduce patients' discomfort, especially the children's one, resulting from the contact of a cold stethoscope head with a child's body.

In an offer of 3M company as given on www.3M website Worldwide: United States:HealthCare there can be found a description of ways for entering laser overprints, limited to 25 characters, on diaphragms and diaphragm of stethoscope heads.

Thus, the to-date used art known arrangements as applied to distinguish the medical stethoscope head of the same kind, result in a uniform and obscure personalization or attempt to positively impress the infant patients. Still, there are no solutions of the medical stethoscope heads of the same kind, that would unambiguously distinguish a single head in the very different ways, depending on personal preferences of a potential user or or in an exact accordance with his/her individual wishes and wishes of the companies which would intend to use them in the advertisement campaigns. The to-date arrangements to distinguish the medical stethoscope head, besides use of the removable decorative covers described in the a.m. publications US2001001188, WO 01/13788 and US2004/0048539, are not suitable for the repeated procedures of cleaning, disinfecting and sterilizing, which are required for that kind of equipment. Generally, manufacturers do not allow either dipping of medical stethoscope heads in any liquid or steam sterilization, and they only recommend an external wiping with alcohol or soap solution in water and using low temperature sterilization with ethylene oxide. This causes many inconveniences. If an accidental wetting of medical stethoscope head occurs, the stethoscope has to be delivered to an authorized service, because its construction does not enable the user to personally dismantle it so as to remove liquid residuals and to properly dry the stethoscope. Medical stethoscope heads have to be periodically cleaned internally by an authorized service from the dirt that may get inside while keeping them in a pocket or during transportation in a medical bag. In case the sterilization is required, the medical stethoscope head has to be subject to a very long and expensive low temperature sterilization processes with ethylene oxide, followed by a 36 hour long ventilation processes in the aeration chambers, which processes are available only in large hospitals.

The prior art includes the medical stethoscope heads with the exchangeable bells, the diaphragm bells inclusive, but these cannot remove the above-mentioned inconveniences completely, because the former are screwed on the threaded pins of stethoscope head body, hence they are not attached to the head's body in an unequivocal, always the same position in relation to axes of their inlet pipes. It disables their proper individualization, in the context of general design rules of designing, by depositing on the heads the individually designed distinguishing graphical and/or structural elements, e.g. such as personalizing structural elements, initials or user's name, logo of a company which uses a stethoscope in advertising campaigns, etc., which should not be located accidentally, but always in the same position in relation to the axis of the inlet pipe, which is often used by designers of personalizing elements as a point of reference for a distribution of individual elements which distinguish a stethoscope head.

Patent US 5,747,752 discloses a medical stethoscope according to the preamble of claim 1.

There is also a market demand for the medical stethoscope heads, provided with the user-distinguishing elements, enabling their users to clean and wash them up and, if the need occurs, could be wholly subject to high temperature sterilization, especially steam sterilization.

Thus, an object of the invention is to provide a medical stethoscope head, a construction of which allows easy, distinct and broadest possible, user distinguishing marking in relation to other medical stethoscope heads of the same kind, manufactured in series by the same manufacturer, and easy identification thereof by the user or a group of users.

Another object of the invention is to provide a medical stethoscope head, the appearance of which would be easily changeable, also at the retail point of sale of sale, or by the stethoscope user by means of the simple and easy accessible tools.

Another object of the invention is to provide a medical stethoscope head, easy identifiable, also at retail point of sale, or by the stethoscope user, in accordance with individual preferences of the users, and adapted to a total dismantle by the user to subject it, both as a whole and in dismantled condition, to cleaning and sterilization, which is obligatory for such kind of medical equipment, also in the commonly accessible table top steam sterilizers.

Another object of the invention is to provide the exchangeable and distinct, distinguishing means for medical stethoscope head, which is easy to mount onto the medical stethoscope head in accordance with individual purchaser's preferences, at retail point of sale or by the user himself.

Further object of the invention is to provide easy exchangeable diaphragm portion for medical stethoscope head, which makes it possible to identify and distinctly personalize stethoscopes, also at retailers, according to preferences and wishing of individual users, and simultaneously it is adapted to be cleaned and sterilized, which is obligatory for that kind of medical equipment, also in high temperature, especially may be sterilized by steam in the commonly available table top steam sterilizers.

The invention's intended purpose is carried out by a medical stethoscope head according to claim 1.

Preferably, on its lower surface said personalizing-identifying ring is provided with at least one positioning member adopted for cooperating with means provided on said diaphragm portion for explicit positioning said personalizing-identifying ring in the preset angular position in relation to said axis of said inlet pipe.

In particular, a fastening element, preferably a thread, catch or recess, is provided on one of said side surfaces of said personalizing-identifying ring to removably secure the said personalizing-identifying ring to the said diaphragm portion.

Also preferably, on one of its upper surface and lower surface, the said personalizing-identifying ring is provided with at least one threaded hole in which a fastening element is situated for securing said personalizing-identifying ring to the diaphragm portion. Especially, said personalizing-identifying ring can be composed of at least two separate members, and also it can be made of any material or set of materials, which are joined with each other in any combination.

Preferably, said fastening ring is elastic and said personalizing-identifying ring is formed integrally with said fastening ring.

In one embodiment, preferably, a fastening element, preferably thread, catch or recess, for connecting said ring with said diaphragm cup is placed on said at least one side surface of said ring.

In another embodiment of the invention, in said lower surface of said ring a hole for placing a screw or a pin, being a fastening element, is provided.

The personalizing-identifying ring may comprise at least two members and, preferably, may be made of any material or set of materials, connected with each other in any combination.

Further, an elastic fastening ring, which is an integral part of the user-distinguishing ring, can be attached to said outer side surface of the user-distinguishing ring.

The diaphragm portion is preferably removably joined to said body by at least one positioning-fastening means to hold the preset angular position of the diaphragm portion in relation to the axis of the inlet pipe of the body.

Said positioning fastening means can be selected from a threaded joint, pin joint, bayonet joint and snap joint.

Preferably in said upper surface of said diaphragm portion an annular recess is formed for placing an identifying-personalizing ring.

The advantage of the subject invention is that it is possible to change an appearance of the medical stethoscope head in a simple manner, either by fastening the exchangeable personalizing-identifying ring having distinguishing means to the upper surface of the diaphragm portion or by removably fastening the exchangeable diaphragm portion provided with the distinguishing means. Such personalization of the medical stethoscope head may be provided not only by manufacturer, but also by the user or some point of service, by using simple tools. A user may select the personalizing-identifying ring (or a diaphragm portion having the distinguishing means) from manufacturer catalogue of the ready-made elements, may request that the additional distinguishing means according to personal preferences be provided or may order manufacturing of the personalizing-identifying ring (or diaphragm portion having distinguishing means) according to his own design using basic materials of stethoscope manufacturer.

It is an advantage of the subject invention that the medical stethoscope head, provided with the personalizing-identifying ring or a diaphragm portion having distinguishing means, makes possible an easy and univocal association of the given stethoscope with its user and allows the user-identification of the particular model of the medical stethoscope head according to personal preferences of its future user on a day of purchase or in the future. When those personal preferences changed, it would be possible to easy swap of the personalizing-identifying ring because the manner of fastening both the personalizing-identifying ring and a diaphragm portion allows an easy swap for another one.

It is an advantage of the subject invention that the number of the user-distinguishing variants of the medical stethoscope head for distinguishing it from other items of the same kind is practically unlimited, because the number of designs of distinguishing means by using any technologies, materials and colours is unlimited.

The above feature ensures to the user of the stethoscope a comfort of distinguishing his/her stethoscope from other items of the same model, satisfies his/her aesthetic and ambition requirements and allows the companies that use stethoscopes in the advertising campaigns, individualizing the medical stethoscope head in such a way that stethoscopes are significantly distinguished from the same models of stethoscopes used in other campaigns.

The considerable advantage of the invention is that the medical stethoscope head according to the invention, can be easily dismantled into individual elements directly by the user, by using the commonly available tools to subject it to the periodic cleaning, washing, disinfections and high temperature sterilization procedures and in table top steam sterilizers commonly available in the physician's offices. The reassembling of the stethoscope head also has not to be made in a specialized service point, because the structure of the head guarantees repeatable locations of all the elements in relation to axis of the body inlet pipe of the medical stethoscope head.

The invention is depicted in the drawing of the embodiments, in which:
Fig. 1 is a perspective view of the first embodiment of the medical stethoscope head having a personalizing-identifying ring according to the invention, mounted therein;
Fig. 2 is a medical stethoscope head according to fig. 1, in a side view and partly in cross section;
Fig. 3 is a view of the medical stethoscope head dismantled into respective parts according to fig. 1 and 2, in perspective view;
Fig. 4 to 7 are cross sections of the subsequent modifications of the first embodiment of the medical stethoscope head, showing the alternative structures of the personalizing-identifying ring and fastening thereof to a diaphragm portion;
Fig. 8 is an embodiment of the user-distinguishing ring, in perspective view, partly in cross section;
Fig. 9 is the second embodiment of the medical stethoscope head having the user-distinguishing element in a form of the diaphragm portion according to invention, in a perspective view;
Fig. 10 to 20 are cross sections of the subsequent modifications of the medical stethoscope head of fig. 9, showing different preferred variants of the positioning-connecting means of the diaphragm portion of a stethoscope head;
Fig. 21 is a perspective view of an exchangeable diaphragm portion for the medical stethoscope head of fig. 9.

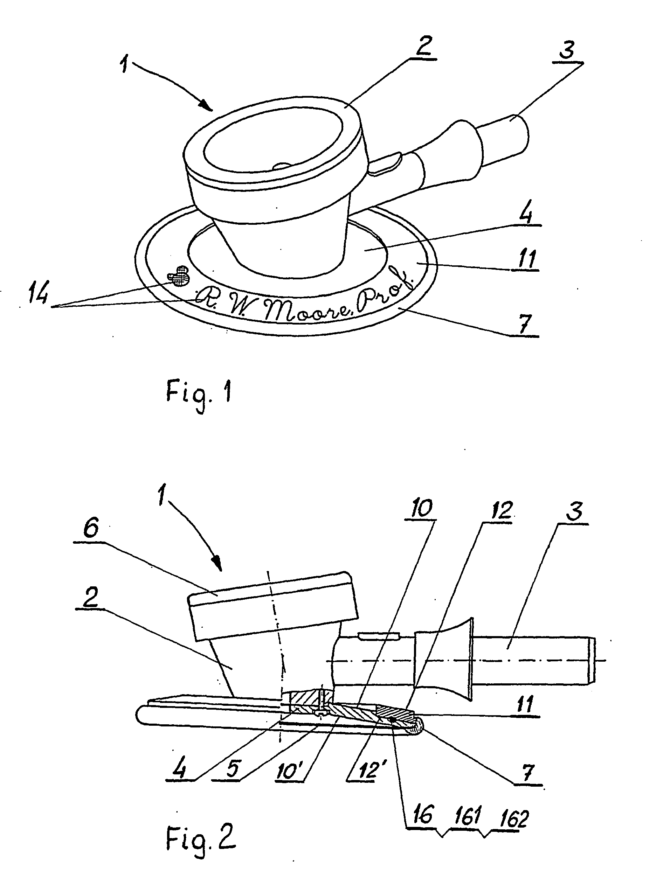

In the preferred embodiment in fig.1-3, a medical stethoscope head comprises a body 2 having an inlet pipe 3 and diaphragm portion 4 with diaphragm 5 (shown in fig. 2-7). In a presented embodiment, the body 2 is provided with insulation ring 6. The diaphragm 5 is hold at a diaphragm portion with fastening ring 7, and inlet pipe is positioned in the body 2 by using a pin 8 and a helical spring 9. The medical stethoscope head 1 may have any shape, such as, for instance, cardiologic, internist, paediatrician medical stethoscope heads, and the like.

According to the invention, at an upper surface 10 of the diaphragm portion 4 of the medical stethoscope head 1, opposite to lower surface 10' adjacent to diaphragm 5, there is mounted a personalizing-identifying ring 11 having an upper surface 12, a lower surface 12', and outer 13 and inner 13' side surfaces. The personalizing-identifying ring is a separate element and is fastened in an optionally selected area of the upper surface 10 of the diaphragm portion 4 in a fixed position in relation to axis 3' of the inlet pipe 3. The personalizing-identifying ring 11 comprises the distinguishing means 14, which specifically may be the distinguishing texture or colour of the whole personalizing-identifying ring 11 and/or different kinds of the user-distinguishing marks located at its upper surface, such as overprints, engraved emblems and inscriptions, decorative elements fastened onto a part or its entire upper surface, a shape of its upper surface and the like. These distinguishing means 14 can be selected individually in accordance with the individual preferences of a stethoscope user and may be produced on the ready-made user-distinguishing rings 11, by the manufacturer of a medical stethoscope head or by the points of sale, by employing the manufacturer's basic elements.

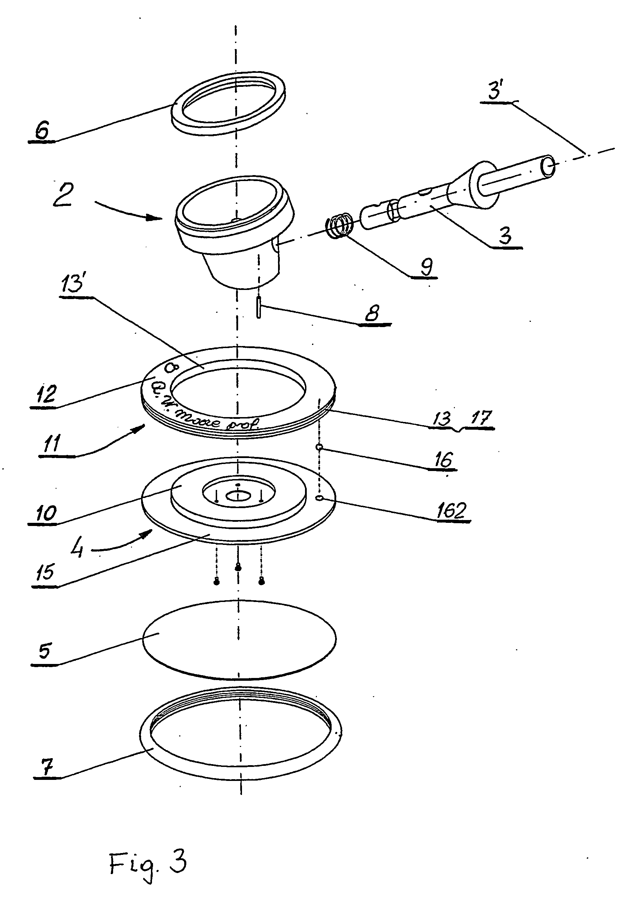

The personalizing-identifying ring 11 in an embodiment in fig. 2, 3 is mounted on the upper surface 10 of the diaphragm portion 4, especially in recess 15 matching by shape and size the shape and size of the personalizing-identifying ring 11 and embracing, wholly or partly, the personalizing-identifying ring 11. In the embodiment of fig. 1-3, the recess 15 extends from a circumferential edge of the diaphragm portion 4 radially to its centre. To set of exact angular position of the personalizing-identifying ring 11 on the diaphragm portion 4 in relation to the axis 3' of the inlet pipe 3, and thus to unequivocally define a position of the distinguishing means 14 on the diaphragm portion 4, the personalizing-identifying ring 11 is provided with at least one location member 16 for placing it in the unequivocally fixed angular position in relation to axis 3' of the inlet pipe 3. In the embodiment in figs. 1-3, the location member 16 is in a form of a metal sphere, a lower part of which is fixed or removable mounted in a seat 162 provided on the upper surface 10 of the diaphragm portion 4, and its upper part is mounted in a seat 161 (shown in fig. 2) provided in the lower surface 12' of the personalizing-identifying ring 11.

In the embodiment of fig. 2, 3, the outer side surface 13 of the personalizing-identifying ring 11 comprises a fastening element 17 having a form of a thread, and the fastening ring 7 having a thread on its internal surface and supporting the diaphragm 5 at the diaphragm portion 4 is screwed on said thread. In such a manner, the personalizing-identifying ring 11 and the diaphragm 5 is simultaneously kept at the diaphragm portion 4.

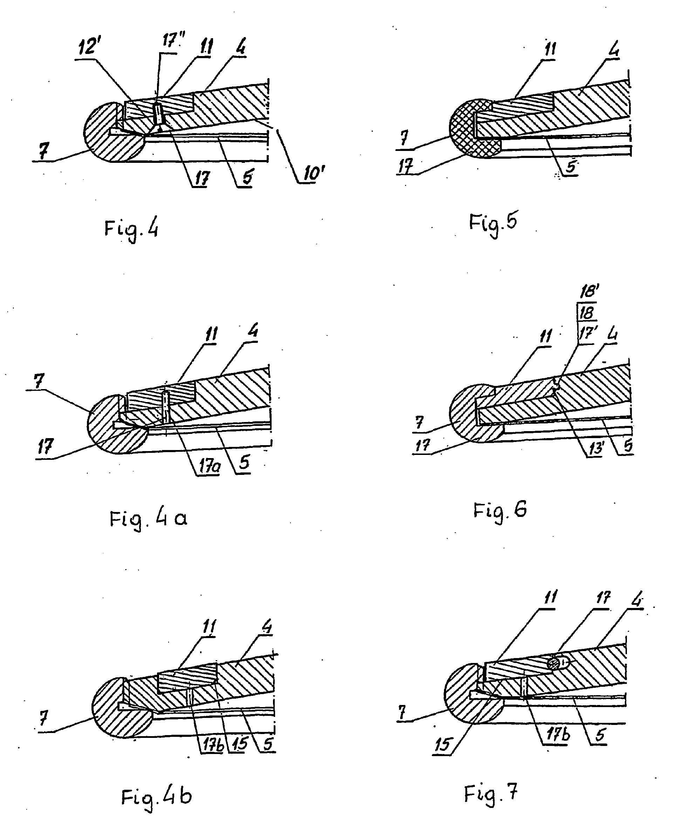

Figs. 4-7 show other preferred variants of fastening the personalizing-identifying ring 11 to the diaphragm portion 4.

In a variant shown in fig. 4, the personalizing-identifying ring 11 is fastened to the diaphragm portion 4 with at least one fastening element 17 having a form of a screw, introduced from underneath to an aperture in the diaphragm portion and screwed into a threaded hole 17" in a lower surface 12' of the personalizing-identifying ring 11. In this case, the screw is simultaneously a location member. In that embodiment, the fastening ring 7 is not connected with the personalizing-identifying ring 11.

In the variant shown in fig. 4a, the personalizing-identifying ring 11 is fastened to the diaphragm portion 4 by at least one fastening element 17 in a form of a pin, which is tightly positioned in a aperture 17a of the diaphragm portion 4 and in a blind hole in a lower surface 12' of the personalizing-identifying ring 11. In this case, a pin being a fastening element 17 is simultaneously, together with the holes, a location member.

In the variant in fig. 4b, the personalizing-identifying ring 11 is fastened, in push-in manner, into a circumferential recess 15 of the diaphragm portion 4 and the circumferential recess 15 is located distally from the diaphragm portion side surface. The diaphragm portion 4 is provided with apertures 17b cooperating with a location member for the personalizing-identifying ring 11 in a suitable angular position in relation to axis 3' of the inlet pipe 3 and serving for introduction of a pin pushing out the personalizing-identifying ring 11, in the case it is to be removed from the diaphragm portion 4.

In the variant shown in fig. 5, the personalizing-identifying ring 11 is fastened to the diaphragm portion 4 by the fastening element 17 in a form of the elastic fastening ring 7, which tightly encloses a lower edge of the diaphragm 5, a side surface of the diaphragm portion 4 and overlaps at upper surface 12 of the personalizing-identifying ring 11. Preferably, the rim of the personalizing-identifying ring 11 is shaped such that it comprises a cut-off in which an upper part of the elastic fastening ring 7 is positioned.

Fig. 6 shows an embodiment of the medical stethoscope head similar to those of fig. 5, an elastic fastening element being connected with the outer side surface of the personalizing-identifying ring 11 and forming an integral part thereof and being simultaneously a fastening ring 7 for a diaphragm 5.

The inner side surface 13' of the personalizing-identifying ring 11 is provided with an additional fastening element 17' in a form of a continuous or point circumferential protrusion 18, located in a groove 18' formed in a side surface of the recess 15 for the personalizing-identifying ring 11 in the diaphragm portion 4. The additional fastening element 17' provides a holding of the personalizing-identifying ring 11 and fastening ring 7 at the diaphragm portion 4.

Fig. 7 presents a subsequent embodiment of the medical stethoscope head, which comprises the personalizing-identifying ring 11 fastened to the diaphragm portion 4 by a fastening element 17 in a form of a spring ring. The spring ring is located in a seat formed between the personalizing-identifying ring 11 and the diaphragm portion 4. The seat for the spring ring is in a form of circumferential groove in an inner side surface 13' of the personalizing-identifying ring 11 and a circumferential groove in a side surface of the recess 15 in the diaphragm portion 4. The outer side surface 13 of the personalizing-identifying ring 11 contacts a fastening ring 7 by which the diaphragm 5 is supported on the diaphragm portion 4. In such an arrangement, the diaphragm portion 4 is provided with at least one aperture 17b, which cooperates with the element positioning the angular position of the personalizing-identifying ring and is made between upper 10 and lower 10' surfaces of the diaphragm portion 4. The aperture 17b allows pushing the personalizing-identifying ring 11 out and separating it from the diaphragm portion 4 as a result of pushing it out with a tool in a form of a pin.

In each case, the personalizing-identifying ring 11 may form one piece or may be sectioned and comprise any number of members, if it is possible to keep them at the surface of the diaphragm portion 4 to form the distinguishing means.

Of course, figs. 1-7's depicted embodiments of the personalizing-identifying ring 11 and its fastening and location members are only exemplary. The skilled one may anticipate many different elements for fastening the personalizing-identifying ring 11 and for fixing a position of the personalizing-identifying ring 11 in relation to axis 3' of the inlet pipe 3 of the medical stethoscope head 1 such as elements of different snap and pin connections and the like. In some specific cases, the personalizing-identifying ring 11 may be glued to the diaphragm portion 4.

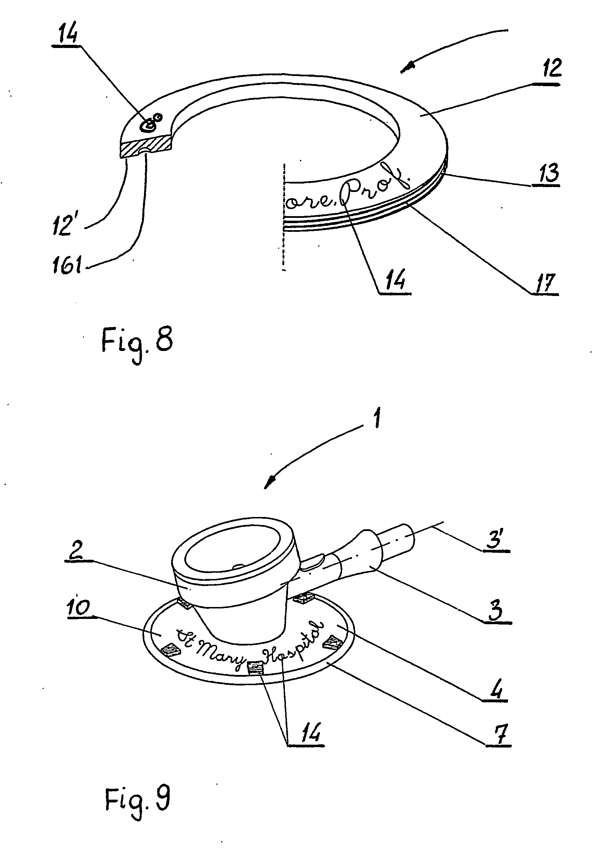

Fig. 8 shows the personalizing-identifying ring 11 according to the invention constituting an enlarged view of the ring described in connection with figs. 2 and 3. The upper surface 12 of the personalizing-identifying ring 11 is provided with, for example, an engraved inscription and set of three decorative hemispheres of different diameters, and at its lower surface 12', designed to contact the diaphragm portion 4, there is at least one location member 16 for an unequivocal location of an angular position of the personalizing-identifying ring 11 in relation to axis 3' of the inlet pipe 3 of the body 2 after mounting the ring in the medical stethoscope head 1. In the embodiment in fig. 8, a location member is a seat 161 formed in a lower surface 12' of the personalizing-identifying ring 11, designed for receiving a sphere 16 (shown in figs. 2 and 3). The outer side surface 13 is provided with a fastening element 17 in a form of a thread for holding the personalizing-identifying ring 11 at the diaphragm portion 4 by a threaded fastening ring 7 for fastening the diaphragm 5.

The personalizing-identifying ring 11 of the medical stethoscope head 1 according to the invention may be designed as described in relation to figs. 4-7. As shown, an element for fastening the personalizing-identifying ring 11 to the diaphragm portion 4 may be, e.g., at least one catch, thread or recess located on side surfaces 13, 13' or a hole for placing screw or pin located on the lower surface 12'.

As shown in fig. 6, an additional elastic ring being an integral part of the personalizing-identifying ring 11 may be attached to its outer side surface 13. The elastic ring functions as an element for fastening the personalizing-identifying ring 11 to the diaphragm portion 4 as well as a fastening ring 7 supporting the diaphragm 5.

The personalizing-identifying ring 11 may be made of any material or combination of materials connected in any combination provided that they meet the requirements for cleaning and sterilizing the medical stethoscope head.

The personalizing-identifying ring 11 may have any width and thickness; it can be made as a single- or multi-layer item, mono-colour or in combination of many colours, of one or more materials that are connected in any combination. Its upper surface 12 may be of any shape. Also, its side surfaces 13 and 13' may be of any shape depending on a variant of its fastening to the diaphragm portion 4.

Fig. 9 shows another embodiment of the medical stethoscope head 1, which, like in the embodiment shown in figs. 1-3, may be of any shape and it comprises the same parts. The difference is that, according to the invention, the medical stethoscope head 1 as presented in fig. 9 comprises the diaphragm portion 4, which at its upper surface 10, opposite to the lower surface 10' adjacent to diaphragm (not shown) has the distinguishing means 14 for distinguishing a medical stethoscope head from all other medical stethoscope heads of that kind. The distinguishing means 14 comprises, for instance, an engraved user name, and a set of the colourful, user-distinguishing decorative elements. Simultaneously, the diaphragm portion 4 is removably joined to a body 2 by at least one positioning-fastening unit 20 allowing a snap of the diaphragm portion 4 and holding the preset angular position of the diaphragm portion 4 in relation to axis 3' of the inlet pipe 3 of the body 1.

An area distinguished by any method, for instance the ones described above in relation to the personalizing-identifying ring 11, may form the distinguishing means 14 of the diaphragm portion 4. The distinguishing means 14 may also be formed by the personalizing-identifying ring 11 mounted in any area at an upper surface 10 of the diaphragm portion 4 and provided with at least one location member to unequivocally settle its angular position in relation to axis 3' of an inlet pipe 3 of a body 2.

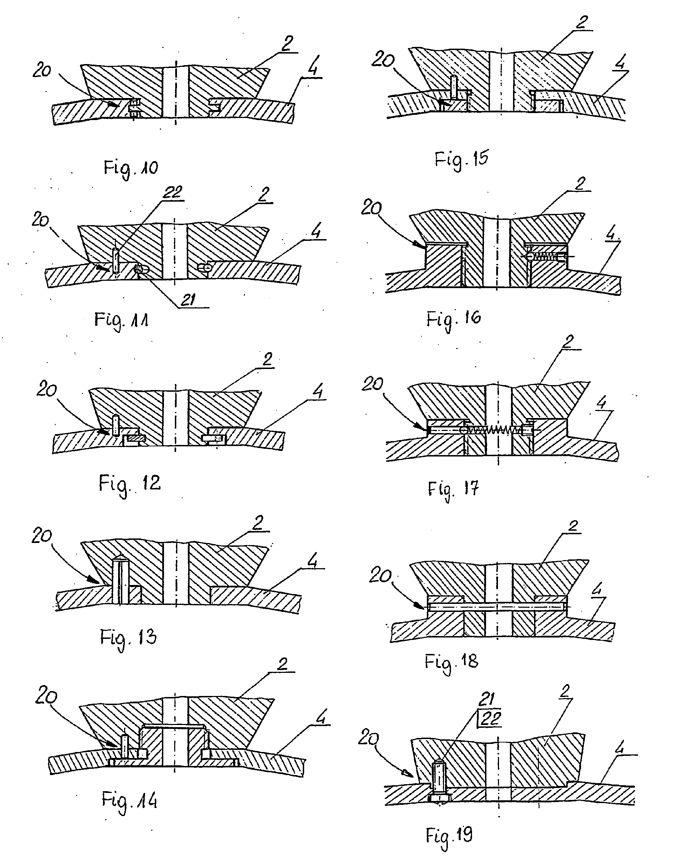

Figs. 10-20 show the different variants of the diaphragm portion 4 and a body 2. The medical stethoscope head of fig. 10 comprises a positioning-connecting means 20 which may comprises a bayonet coupling.

The medical stethoscope head in fig. 11 comprises a positioning-connecting means 20 comprising a fastening circumferential spring ring, which is placed partly in the joining element 21, in the form of a groove, of the diaphragm portion 4 and partly in the joining element, in the form of the groove, of the body 2. The positioning-connecting means 20 comprises also at least one axially positioned location pin positioned in the location member 22, in the form of an aperture, of the diaphragm portion 4.

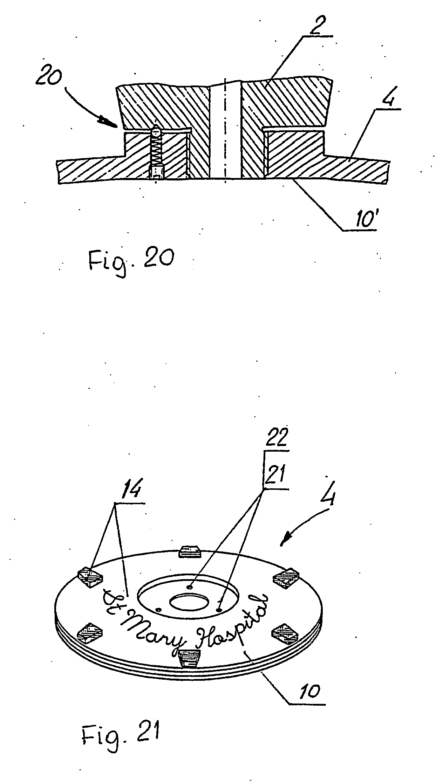

The medical stethoscope head in fig. 12 has a positioning-connecting means 20 comprising a fastening circumferential locking ring and at least one axial positioning pin. The medical stethoscope head from fig. 13 comprises a positioning-connecting means 20 in form of at least one an axially positioned expanding pin. The medical stethoscope head of fig. 14 has a positioning-connecting means 20 comprising a positioned centrally bolt, which is axially fastened and at least one axial positioning pin. The medical stethoscope head shown in fig. 15 comprises a positioning-connecting means 20 in a form of a nut, which is mounted on a threaded lower pin of a body 2 and at least one axial positioning pin. The medical stethoscope head shown in fig. 16 comprises positioning-connecting means 20 having a threaded joint located between the diaphragm portion 4 and a body 2 and a catch, which is situated between the sides of the diaphragm portion 4 and a body 2. The medical stethoscope head from fig. 17 comprises positioning-connecting means 20 similar to that from fig. 16, but in that case, a catch extends along full diameter of the threaded part of a body 2. The medical stethoscope head from fig. 18 comprises positioning-connecting means 20 having a pin, which extends transversally by a protrusion of the diaphragm portion 4 and a body 2. The medical stethoscope head from fig. 19 comprises, especially preferably, a positioning-connecting means 20, the same as shown also in figs. 2 and 3, comprising axially positioned screws, which pass through the aperture, constitute joining elements 21 and location members 22 and formed in the diaphragm portion 4, and screwed into a body 2. The medical stethoscope head from fig. 20 comprises a positioning-connecting means 20 providing with a screwed joint between the diaphragm portion 4 and a body 2 and a catch positioned axially between the diaphragm portion 4 and a body 2.

The arrangements of positioning-connecting means 20 presented above do not exhaust all the possible structures, which are evident to the skilled persons. Any suitable mechanic structures can be used provided that they unequivocally and repeatedly locate the diaphragm portion 4 in a preset angular position in relation to axis 3' of the inlet pipe 3, allow to removably fasten the diaphragm portion 4 to a body 2 and to give a possibility for exchanging the diaphragm portion 4 by simple and easy available tools such that the exchange could be made in a retail point and by the user himself.

Fig. 21 shows one of the possible embodiments of the exchangeable diaphragm portion for a medical stethoscope head. The diaphragm portion 4 comprises a concave lower surface 10', to which a diaphragm is fasten, and an opposite upper surface 10 provided with a distinguishing means 14, which is composed of, for instance, a user-presenting inscription made by any method, and set of identical decorating-user-distinguishing elements made of material different from the remaining part of the diaphragm portion. The diaphragm portion includes three apertures which constitute joining elements 21 for the screws, the same as in fig. 19, which are designed both to fasten the diaphragm portion 4 to a body 2 and to unequivocally settle the diaphragm portion 4 in a preset angular position in relation to axel 3' of the inlet pipe 3 of a body 2.

For instance, the distinguishing means 14 of the diaphragm portion is an area distinguished by any elements and any method, as described in the reference to a user-distinguishing ring.

For instance, as an joining element 21 of the diaphragm portion 4 for attachment can be a hole for piacing screws (best visible in tig. 19), a groove for placing a spring ring (shown in fig. 11), and a thread at surface of central hole in the diaphragm portion (fig. 15) and the like. A location member 22 of the diaphragm portion 4 for establishing position can be in form of a hole for placing screw (fig. 19) or pin (fig. 11), a hole for placing a catch (fig. 17), positioning protrusions, etc.

At an outer surface of the diaphragm portion there is normally a thread for screwing a ring 7 on for fastening a diaphragm 5 or a circumferential recess, which is in a form of a catch for fastening of elastic ring for retaining a diaphragm.

In the especially preferred embodiment, on the upper surface of the diaphragm portion 4 there is an annular recess for positioning therein the personalizing-identifying ring 11. The diaphragm portion 4 may comprise one or more layers; it can be made of one or more materials connected in any combination. The shape of its upper surface 10 may be optional.

The invention was described with reference to the preferred embodiments that should not be treated in a limited sense. The different modifications and variants of the presented arrangements are possible, provided that they are included in the attached claims.

## Claims

1. A medical stethoscope head (1) comprising:
a body (2);
an inlet pipe (3) extending from said body (2) and having an axis (3');
a diaphragm portion (4) having an upper surface (10) and a lower surface (10') opposite to said upper surface (10);
a diaphragm (5) held at said lower surface (10') of said diaphragm portion (4) by a fastening ring (7); wherein the medical stethoscope head can be easily dismantled into individual elements to subject it to periodical cleaning, washing and high temperature sterilization;
said stethoscope head **characterised in that** it comprises:
at least one personalizing-identifying ring (11) for distinguishing and identifying the stethoscope head, said personalizing-identifying ring (11) having an upper surface (12), a lower surface (12'), an outer side surface (13), an inner side surface (13') and said personalizing-identifying ring (11) located with its lower surface (12') on said upper surface (10) of said diaphragm portion (4) in preset angular position in relation to said axis (3') of said inlet pipe (3);
distinguishing means (14) for personalizing and identifying the stethoscope head provided on said upper surface (12) of said at least one personalizing-identifying ring (11).

2. A medical stethoscope head according to claim 1, **characterised in that** on its lower surface (12') said personalizing-identifying ring (11) is provided with at least one positioning member (16) adopted for cooperating with means provided on said diaphragm portion (4) for explicit positioning said personalizing-identifying ring (11) in the preset angular position in relation to said axis (3') of said inlet pipe (3).

3. A medical stethoscope head according to claim 1 or 2, **characterised in that** a fastening element (17), preferably a thread, catch or recess, is provided on one of said side surface (13, 13') of said personalizing-identifying ring (11) for removably securing said personalizing-identifying ring (11) to said diaphragm portion (4).

4. A medical stethoscope head according to claim 1 or 2, **characterised in that** on one of its upper surface (12) and lower surface (12') said personalizing-identifying ring (11) is provided with at least one threaded hole (17") in which a fastening element (17) is situated for securing said personalizing-identifying ring (11) to the diaphragm portion (4).

5. A medical stethoscope head according to claim 1 or 2, **characterised in that** said personalizing-identifying ring (11) is composed of at least two separate members.

6. A medical stethoscope head according to claim 1, **characterised in that** said personalizing-identifying ring (11) is made of any material or set of materials, which are joined each other in any combination.

7. A medical stethoscope head according to claim 1, **characterised in that** said fastening ring (7) is elastic and said personalizing-identifying ring (11) is formed integrally with said fastening ring (7).

8. A medical stethoscope head according to claim 1, **characterized in that** the diaphragm portion (4) is removably joined to said body (2) by at least one positioning-fastening means (20) to hold the preset angular position of the diaphragm portion (4) in relation to the axis (3') of the inlet pipe (3) of the body (2).

9. A medical stethoscope head according to claim 8, **characterized in that** said positioning-fastening means (20) is selected from a threaded joint, pin joint, bayonet joint and snap joint.

10. A medical stethoscope head according to claim 1, **characterized in that** in said upper surface (10) of said diaphragm portion an annular recess (15) is formed for placing the personalizing-identifying ring (11).

## Patentansprüche

1. Stethoskopkopf (1) umfassend:
ein Körper (2);
ein aus dem Körper (2) hinausragendes und eine Achse (3') aufweisendes Einlaufrohr (3);
einen eine Oberfläche (10) und eine dieser gegenüberliegende Unterfläche (10') aufweisenden Abhörteil (4);
eine mittels eines Halterings (7) an der genannten Unterfläche (10') des Abhörteils (4) gehaltene Membran (5), wobei der Stethoskopkopf (1) ist in einzelne Bestandteile zum periodischen Reinigen, Waschen und Hochtemperatursterilisieren einfach zerlegbar,
**dadurch gekennzeichnet, dass**
er mindestens einen Identifizierungs- und Personalisierungsring (11) zur Individualisierung und Personalisierung des Stethoskopkopfs umfasst, welcher Identifizierungs- und Personalisierungsring (11) eine Oberseite (12), eine Unterseite (12'), eine Aussenfläche (13) und eine Innenfläche (13') aufweist und mit seiner Unterseite (12') auf der Oberfläche (10) des Abhörteils (4) in einer vorbestimmten Winkelstellung bezüglich der Einlaufrohrachse (3') angeordnet ist, und
Auszeichnungsmittel (14) zur Individualisierung und Personalisierung des Stethoskopkopfs umfasst, die auf der Oberseite (12) des mindestens einen Identifizierungs- und Personalisierungsrings (11) angeordnet sind.

2. Stethoskopkopf (1) nach Anspruch 1, **dadurch gekennzeichnet, dass** der Identifizierungs- und Personalisierungsring (11) auf seiner Unterseite (12') mit mindestens einem zum Zusammenwirken mit den auf dem Abhörteil (4) angeordneten Mitteln zur Positionierung des Identifizierungs- und Personalisierungsrings (11) in der vorbestimmten Winkelstellung bezüglich der Einlaufrohrachse (3') ausgelegten Positionerungelement (16) versehen ist.

3. Stethoskopkopf (1) nach Anspruch 1 oder 2, **dadurch gekennzeichnet, dass** auf einer der Seitenflächen (13, 13') des Identifizierungs- und Personalisierungsrings (11) ein Befestigungselement (17), vorzugsweise ein Gewinde, ein Rastelement oder eine Vertiefung, zur lösbaren Befestigung des Identifizierungs- und Personalisierungsrings (11) an dem Abhörteil (4) angeordnet ist.

4. Stethoskopkopf (1) nach Anspruch 1 oder 2, **dadurch gekennzeichnet, dass** der Identifizierungs- und Personalisierungsring (11) auf seiner Oberseite (12) oder seiner Unterseite (12') mit mindestens einer Gewindeöffnung (17") versehen ist, in der das Befestigungselement (17) zur lösbaren Befestigung des Identifizierungs- und Personalisierungsrings (11) an dem Abhörteil (4) angeordnet ist.

5. Stethoskopkopf (1) nach Anspruch 1 oder 2, **dadurch gekennzeichnet, dass** der Identifizierungs- und Personalisierungsring (11) aus mindestens zwei separaten Bauteilen besteht.

6. Stethoskopkopf (1) nach Anspruch 1, **dadurch gekennzeichnet, dass** der Identifizierungs- und Personalisierungsring (11) aus einem beliebigen Material oder aus einer beliebigen Materialkombination gefertigt ist.

7. Stethoskopkopf (1) nach Anspruch 1, **dadurch gekennzeichnet, dass** der Haltering (7) elastisch ist und der Identifizierungs- und Personalisierungsring (11) integral mit dem Haltering (7) ausgebildet ist.

8. Stethoskopkopf (1) nach Anspruch 1, **dadurch gekennzeichnet, dass** der Abhörteil (4) mit dem Körper (2) mit Hilfe von Halte- und Befestigungsmitteln (20) zum Aufrechterhalten der vorbestimmten Winkelstellung des Abhörteils (4) bezüglich der Einlaufrohrachse (3') des Körpers lösbar verbunden ist.

9. Stethoskopkopf (1) nach Anspruch 8, **dadurch gekennzeichnet, dass** die Halte- und Befestigungsmitteln (20) aus einer Schrauben-, einer Stift-, einer Bajonett- und einer Rastverbindung gewählt sind.

10. Stethoskopkopf (1) nach Anspruch 1, **dadurch gekennzeichnet, dass** in der Oberseite (10) der Abhörmuschel eine ringförmige Vertiefung (15) zur Aufnahme des Personalisierungsrings (11) ausgebildet ist.

## Revendications

1. Tête (1) de stéthoscope médical, comprenant :
un corps (2);
un tube d'entrée (3) saillant du corps (2) et ayant un axe (3');
un élément auscultatoire (4) ayant une surface supérieure (10) et une surface inférieure (10') opposée à la surface supérieure (10);
une membrane (5) maintenue près de ladite surface inférieure (10') dudit élément auscultatoire (4) à l'aide d'un collier de fixation (7); la tête (1) de stéthoscope médical (1) pouvant être facilement démontable en éléments composants en vue de son nettoyage, son lavage et sa stérilisation périodiques en haute température,
tête de stéthoscope **caractérisée en ce qu'**elle comprend:
au moins une bague d'identification-d'individualisation (11) pour identifier et distinguer la tête de stéthoscope; cette bague (11) a une surface supérieure (12), une surface inférieure (12'), une surface externe (13), une surface interne (13') et ladite bague d'identification-d'individualisation (11) est posée par sa surface inférieure (12') sur la surface supérieure (10) de l'élément auscultatoire (4) dans une position angulaire prédéterminée relativement audit axe (3') du tube d'entrée (3);
des moyens de distinction (14) pour individualiser et identifier la tête de stéthoscope, se trouvant sur ladite surface supérieure (12) d'au moins une ladite bague d'identification- d'individualisation (11).

2. Tête de stéthoscope médical selon la revendication 1, **caractérisée en ce que** sur sa surface inférieure (12') ladite bague d'identification-d'individualisation (11) est munie d'au moins un élément de fixation (16) adapté à coopérer avec les moyens qui se trouvent sur ledit élément auscultatoire (4) pour une localisation précise de ladite bague d'identification-d'individualisation (11) dans la position angulaire prédéterminée relativement à l'axe (3') du tube d'entrée (3).

3. Tête de stéthoscope médical selon la revendication 1 ou 2, **caractérisée en ce que** l'élément de fixation (17), avantageusement un filetage, un crochet ou un creux, est assuré sur une face de ladite surface latérale (13, 13') de ladite bague d'identification-d'individualisation (11) pour une fixation de façon amovible de ladite bague d'identification-d'individualisation (11) audit élément auscultatoire (4).

4. Tête de stéthoscope médical selon la revendication 1 ou 2, **caractérisée en ce que** sur une de ses surfaces supérieure (12) ou inférieure (12') ladite bague d'identification-d'individualisation (11) est munie d'au moins une ouverture filetée (17"), dans laquelle est introduit l'élément de fixation (17) pour la fixation de ladite bague d'identification- d'individualisation (11) audit élément auscultatoire (4).

5. Tête de stéthoscope médical selon la revendication 1 ou 2, **caractérisée en ce que** ladite bague d'identification-d'individualisation (11) se compose d'au moins deux éléments indépendants.

6. Tête de stéthoscope médical selon la revendication 1, **caractérisée en ce que** ladite bague d'identification-d'individualisation est realisée en une matière quelconque ou en ensemble de matières liées entre elles selon une combinaison quelconque.

7. Tête de stéthoscope médical selon la revendication 1, **caractérisée en ce que** ledit collier de fixation (7) est flexible, et ladite bague d'identification-d'individualisation (11) est intégrale avec ledit collier de fixation (7).

8. Tête de stéthoscope médical selon la revendication 1, **caractérisée en ce que** ledit élément auscultatoire (4) est fixée de façon amovible audit corps (2) à l'aide d'au moins un moyen de stabilisation de fixation (20) en vue du maintien de la position angulaire prédéterminée dudit élément auscultatoire (4) relativement à l'axe (3') du tube d'entrée (3) du corps.

9. Tête de stéthoscope médical selon la revendication 8, **caractérisée en ce que** lesdits moyens de stabilisation de fixation (20) sont choisis de parmi un joint à filetage, à goupille, à baïonette et à verrou.

10. Tête de stéthoscope médical selon la revendication 1, **caractérisée en ce que** dans ladite surface supérieure (10) dudit élément auscultatoire (4) est formé un creux annulaire (15) pour introduire ladite bague d'identification- d'individualisation (11).
